# EUROPEAN PATENT APPLICATION

(11) **EP 2 329 845 A1**
(43) Date of publication of application: **08.06.2011**
(21) Application number: 09808225.8
(22) Date of filing: 13.08.2009
(51) Int. Cl.: A61K 39/145, A61K 39/39, A61P 31/16

(54) **AVIAN INFLUENZA VIRUS ANTIGEN, AND BOOSTER IMMUNIZATION METHOD FOR AVIAN INFLUENZA VACCINE IN COMBINATION WITH MUCOSAL ADJUVANT WHICH IS EFFECTIVE THROUGH ORAL ADMINISTRATION**

(30) Priority: 18.08.2008 JP 2008210021
(71) Applicant: The Kitasato Institute, Tokyo 108-8641 (JP)
(72) Inventor: MIYOSHI, Yukihiro, Kitamoto-shi Saitama 364-0026 (JP); ITO, Akira, Kitamoto-shi Saitama 364-0026 (JP); SUSA, Kentaro, Kitamoto-shi Saitama 364-0026 (JP); HIMENO, Naomi, Kitamoto-shi Saitama 364-0026 (JP); GOTANDA, Toru, Kitamoto-shi Saitama 364-0026 (JP)
(74) Representative: Bösl, Raphael Konrad
(86) International application number: PCT/JP2009/064285
(87) International publication number: WO 2010/021289

(57) **Abstract**

When ELISA antibody titer dropped in chickens intramuscularly inoculated with an oil vaccine, recombinant HA and NP antigens prepared using gene recombinant technology were orally administered in combination with synthetic oligo CpG as a mucosal adjuvant to the chickens. Even when the immune response induced with the oil vaccine was impaired, the antibody titer was re-induced by orally administering the recombinant HA and NP antigens, and synthetic CpG-ODN in combination.

SEQUENCE LISTING

<110>  THE KITASATO INSTITUTE

<120>  AVIAN INFLUENZA VIRUS ANTIGEN, AND BOOSTER IMMUNIZATION METHOD FOR AVIAN
INFLUENZA VACCINE IN COMBINATION WITH MUCOSAL ADJUVANT WHICH IS EFFECTIVE THROUGH
ORAL ADMINISTRATION

<130>  K4-A0802P

<150>  JP 2008-210021
<151>  2008-08-18

<160>  9

<170>  PatentIn version 3.4

<210>  1
<211>  1539
<212>  DNA
<213>  Influenza A virus

<400>  1
atggaccaaa tttgcattgg ttaccatgca aacaactcaa cagagcaggt tgacacaata      60

atggaaaaga atgttactgt cacgcatgcc caagacatac tggaaaagac acacaatggg     120

aagctctgca gtctaaatgg agttaagcct ctcattttga gggattgtag tgtagctgga     180

tggctcctcg gaaaccccat gtgtgatgaa ttcctcaatg tgccggaatg gtcttacata     240

gtggagaagg acagcccaat caatggcctc tgctacccag gggatttcaa cgactatgaa     300

gagctgaaac acctgttgag cagtacgaac cattttgaga aaattcaaat catccccagg     360

agttcttggt ccaatcatga tgcctcatca ggagtgagct ccgcatgtcc atataatggg     420

aggtcctcct ttttcagaaa tgtagtgtgg ctcatcaaaa agaacaatgc atacccaaca 480

ataaaaagga attacaataa tactaaccaa gaagatcttt tggtactgtg ggggattcac 540

catcctaatg atgcaacaga gcagacaaag ctctatcaaa acccaaccac ctatgtttct 600

gttggaacat caacactgaa ccagagatcg gtcccagaaa tagctaccag gcccaaagta 660

aatgggcaaa gtggaagaat agagtttttc tggacaatct taaagccaaa tgatgccatc 720

aatttcgaga gtaatggaaa ttttattgct ccagaatatg catacaaaat tgccaagaaa 780

ggagactcag caatcatgaa aagtggattg gagtatggta accgcaacac caagtgtcaa 840

actccaatgg gtgcaataaa ctccagcatg ccatttcaca acatacaccc tctcaccatt 900

ggggaatgcc ccagatacgt gaagtcagat agattagtcc ttgcaacagg gctcaggaat 960

gtccctcaaa gagaaacaag aggactattt ggggccatag caggcttcat agaaggaggg 1020

tggcaaggaa tggtagacgg ttggtatgga taccaccata gcaacgagca agggagtgga 1080

tacgctgcag acaaagagtc cactcaaaag gcaatagatg gaatcactaa taaggtcaac 1140

tcaatcattg acaaaatgaa cactcagttt gaggccgttg gaaaggaatt taataactta 1200

gaaaggagga tagagaattt gaacaagaaa atggaagacg gattcctaga tgtctggact 1260

tataatgctg aacttctggt tctcatggaa agtgagagaa ccctagactt tcatgactca 1320

aatgtcaaga acctttacga caaggttcga ctacagctta gggataatgc aaaggagctg 1380

ggtaatggtt gtttcgagtt ctatcacaaa tgtgatgatg aatgtatgga aagtgtaaga 1440

aacggaacgt atgactaccc gcagtattca gaagaggcaa gactaaacag agaggaaata 1500

agtggagtaa aattggaatc aataggaact taccaaata                                1539

<210>  2
<211>  513
<212>  PRT
<213>  Influenza A virus

<400>  2

Met Asp Gln Ile Cys Ile Gly Tyr His Ala Asn Asn Ser Thr Glu Gln
1               5                   10                  15

Val Asp Thr Ile Met Glu Lys Asn Val Thr Val Thr His Ala Gln Asp
            20                  25                  30

Ile Leu Glu Lys Thr His Asn Gly Lys Leu Cys Ser Leu Asn Gly Val
        35                  40                  45

Lys Pro Leu Ile Leu Arg Asp Cys Ser Val Ala Gly Trp Leu Leu Gly
    50                  55                  60

Asn Pro Met Cys Asp Glu Phe Leu Asn Val Pro Glu Trp Ser Tyr Ile
65                  70                  75                  80

Val Glu Lys Asp Ser Pro Ile Asn Gly Leu Cys Tyr Pro Gly Asp Phe
                85                  90                  95

Asn Asp Tyr Glu Glu Leu Lys His Leu Leu Ser Ser Thr Asn His Phe

Arg 100 105 110
Glu Lys Ile Gln Ile Ile Pro Arg Ser Ser Trp Ser Asn His Asp Ala
115 120 125
Ser Ser Gly Val Ser Ser Ala Cys Pro Tyr Asn Gly Arg Ser Ser Phe
130 135 140
Phe Arg Asn Val Val Trp Leu Ile Lys Lys Asn Asn Ala Tyr Pro Thr
145 150 155 160
Ile Lys Arg Asn Tyr Asn Asn Thr Asn Gln Glu Asp Leu Leu Val Leu
165 170 175
Trp Gly Ile His His Pro Asn Asp Ala Thr Glu Gln Thr Lys Leu Tyr
180 185 190
Gln Asn Pro Thr Thr Tyr Val Ser Val Gly Thr Ser Thr Leu Asn Gln
195 200 205
Arg Ser Val Pro Glu Ile Ala Thr Arg Pro Lys Val Asn Gly Gln Ser
210 215 220
Gly Arg Ile Glu Phe Phe Trp Thr Ile Leu Lys Pro Asn Asp Ala Ile
225 230 235 240
Asn Phe Glu Ser Asn Gly Asn Phe Ile Ala Pro Glu Tyr Ala Tyr Lys

245 250 255

Ile Ala Lys Lys Gly Asp Ser Ala Ile Met Lys Ser Gly Leu Glu Tyr
260 265 270

Gly Asn Arg Asn Thr Lys Cys Gln Thr Pro Met Gly Ala Ile Asn Ser
275 280 285

Ser Met Pro Phe His Asn Ile His Pro Leu Thr Ile Gly Glu Cys Pro
290 295 300

Arg Tyr Val Lys Ser Asp Arg Leu Val Leu Ala Thr Gly Leu Arg Asn
305 310 315 320

Val Pro Gln Arg Glu Thr Arg Gly Leu Phe Gly Ala Ile Ala Gly Phe
325 330 335

Ile Glu Gly Gly Trp Gln Gly Met Val Asp Gly Trp Tyr Gly Tyr His
340 345 350

His Ser Asn Glu Gln Gly Ser Gly Tyr Ala Ala Asp Lys Glu Ser Thr
355 360 365

Gln Lys Ala Ile Asp Gly Ile Thr Asn Lys Val Asn Ser Ile Ile Asp
370 375 380

Lys Met Asn Thr Gln Phe Glu Ala Val Gly Lys Glu Phe Asn Asn Leu

385                 390                 395                 400

Glu Arg Arg Ile Glu Asn Leu Asn Lys Lys Met Glu Asp Gly Phe Leu
                405                 410                 415

Asp Val Trp Thr Tyr Asn Ala Glu Leu Leu Val Leu Met Glu Ser Glu
            420                 425                 430

Arg Thr Leu Asp Phe His Asp Ser Asn Val Lys Asn Leu Tyr Asp Lys
        435                 440                 445

Val Arg Leu Gln Leu Arg Asp Asn Ala Lys Glu Leu Gly Asn Gly Cys
    450                 455                 460

Phe Glu Phe Tyr His Lys Cys Asp Asp Glu Cys Met Glu Ser Val Arg
465                 470                 475                 480

Asn Gly Thr Tyr Asp Tyr Pro Gln Tyr Ser Glu Glu Ala Arg Leu Asn
                485                 490                 495

Arg Glu Glu Ile Ser Gly Val Lys Leu Glu Ser Ile Gly Thr Tyr Gln
            500                 505                 510

Ile

<210>  3

<211>  1482
<212>  DNA
<213>  Influenza A virus

<400>  3
gcgcttcaag gcactaaacg atcttatgaa cagatggaaa ctggtggaga acgccagaat      60

gccactgaga tcagagcatc tgttggaaga atggttggtg gaattgggag gttttacata     120

cagatgtgca ctgagctcaa actcagcgac tatgaaggaa ggctgattca gaacagcata     180

acaatagaga gaatggtcct ctctgcattt gatgaaagga ggaacaaata cctggaagaa     240

catcccagtg cggggaagga cccgaagaaa actggaggtc caatttatcg aaggagagac     300

gggaaatgga tgagagagct gattctatat gacaaagagg aaatcaggag gatctggcgt     360

caagcgaaca atggagaaga cgcgactgct ggtctcactc acctgatgat ctggcattcc     420

aatctaaatg atgccacata ccagagaaca agagctcttg tgcgtactgg aatggacccc     480

agaatgtgct ctctgatgca aggatcaact ctcccgagga gatctggagc tgctggtgca     540

gcagtaaagg gagtcggaac gatggtgatg gaactaattc ggatgataaa acgagggatc     600

aatgatcgga atttctggag aggcgaaaat ggacgaagaa caaggattgc atatgagaga     660

atgtgcaaca tcctcaaagg gaaattccaa acagcagcac aaagagcaat gatggaccag     720

gtgcgagaaa gcagaaatcc tgggaatgct gaaatcgaag atctcatctt cctggcacgg     780

tctgcactca tcctgagagg atcagtggcc cataagtcct gcttgcctgc gtgtgtgtac     840

ggacttgctg tggccagtgg atatgacttt gagagagaag ggtactctct agtcggaata     900

gacccttttc gtctgcttca aaacagccag gtcttcagtc tcattagacc aaatgagaat     960

ccagcacaca agagtcaatt ggtgtggatg gcatgccatt ctgcagcatt tgaagacctg 1020

agagtctcaa gtttcatcag agggacaaga gtagtcccaa gaggacaact atccaccaga 1080

ggagttcaaa ttgcttcaaa tgagaacatg gaaacaatgg actccagcac tcttgaactg 1140

aggagcagat attgggctat aaggaccagg agtggaggaa acaccaatca acagagagca 1200

tctgcaggac aaatcagtgt acagcctact ttctcggtac agagaaatct tcccttcgaa 1260

agagcgacca ttatggcggc gttcacaggg aatactgagg gcagaacatc tgacatgagg 1320

actgaaatca taaggatgat ggaaagtgcc agaccagagg atgtgtcttt ccaggggcgg 1380

ggagtcttcg agctctcgga cgaaaaggca acgaacccga tcgtgccttc ctttgacatg 1440

agtaatgaag gatcttattt cttcggagac aatgcagagg ag 1482

<210> 4
<211> 494
<212> PRT
<213> Influenza A virus

<400> 4

Ala Leu Gln Gly Thr Lys Arg Ser Tyr Glu Gln Met Glu Thr Gly Gly
1               5                   10                  15

Glu Arg Gln Asn Ala Thr Glu Ile Arg Ala Ser Val Gly Arg Met Val
            20                  25                  30

Gly Gly Ile Gly Arg Phe Tyr Ile Gln Met Cys Thr Glu Leu Lys Leu

        35                  40                  45

Ser Asp Tyr Glu Gly Arg Leu Ile Gln Asn Ser Ile Thr Ile Glu Arg
    50                  55                  60

Met Val Leu Ser Ala Phe Asp Glu Arg Arg Asn Lys Tyr Leu Glu Glu
65                  70                  75                  80

His Pro Ser Ala Gly Lys Asp Pro Lys Lys Thr Gly Gly Pro Ile Tyr
                85                  90                  95

Arg Arg Arg Asp Gly Lys Trp Met Arg Glu Leu Ile Leu Tyr Asp Lys
            100                 105                 110

Glu Glu Ile Arg Arg Ile Trp Arg Gln Ala Asn Asn Gly Glu Asp Ala
        115                 120                 125

Thr Ala Gly Leu Thr His Leu Met Ile Trp His Ser Asn Leu Asn Asp
    130                 135                 140

Ala Thr Tyr Gln Arg Thr Arg Ala Leu Val Arg Thr Gly Met Asp Pro
145                 150                 155                 160

Arg Met Cys Ser Leu Met Gln Gly Ser Thr Leu Pro Arg Arg Ser Gly
                165                 170                 175

Ala Ala Gly Ala Ala Val Lys Gly Val Gly Thr Met Val Met Glu Leu

            180                 185                 190

Ile Arg Met Ile Lys Arg Gly Ile Asn Asp Arg Asn Phe Trp Arg Gly
        195                 200                 205

Glu Asn Gly Arg Arg Thr Arg Ile Ala Tyr Glu Arg Met Cys Asn Ile
    210                 215                 220

Leu Lys Gly Lys Phe Gln Thr Ala Ala Gln Arg Ala Met Met Asp Gln
225                 230                 235                 240

Val Arg Glu Ser Arg Asn Pro Gly Asn Ala Glu Ile Glu Asp Leu Ile
                245                 250                 255

Phe Leu Ala Arg Ser Ala Leu Ile Leu Arg Gly Ser Val Ala His Lys
            260                 265                 270

Ser Cys Leu Pro Ala Cys Val Tyr Gly Leu Ala Val Ala Ser Gly Tyr
        275                 280                 285

Asp Phe Glu Arg Glu Gly Tyr Ser Leu Val Gly Ile Asp Pro Phe Arg
    290                 295                 300

Leu Leu Gln Asn Ser Gln Val Phe Ser Leu Ile Arg Pro Asn Glu Asn
305                 310                 315                 320

Pro Ala His Lys Ser Gln Leu Val Trp Met Ala Cys His Ser Ala Ala

                325                 330                 335

Phe Glu Asp Leu Arg Val Ser Ser Phe Ile Arg Gly Thr Arg Val Val
            340                 345                 350

Pro Arg Gly Gln Leu Ser Thr Arg Gly Val Gln Ile Ala Ser Asn Glu
        355                 360                 365

Asn Met Glu Thr Met Asp Ser Ser Thr Leu Glu Leu Arg Ser Arg Tyr
    370                 375                 380

Trp Ala Ile Arg Thr Arg Ser Gly Gly Asn Thr Asn Gln Gln Arg Ala
385                 390                 395                 400

Ser Ala Gly Gln Ile Ser Val Gln Pro Thr Phe Ser Val Gln Arg Asn
                405                 410                 415

Leu Pro Phe Glu Arg Ala Thr Ile Met Ala Ala Phe Thr Gly Asn Thr
            420                 425                 430

Glu Gly Arg Thr Ser Asp Met Arg Thr Glu Ile Ile Arg Met Met Glu
        435                 440                 445

Ser Ala Arg Pro Glu Asp Val Ser Phe Gln Gly Arg Gly Val Phe Glu
    450                 455                 460

Leu Ser Asp Glu Lys Ala Thr Asn Pro Ile Val Pro Ser Phe Asp Met

465                 470                 475                 480

Ser Asn Glu Gly Ser Tyr Phe Phe Gly Asp Asn Ala Glu Glu
                485                 490

<210>  5
<211>  31
<212>  DNA
<213>  Artificial

<220>
<223>  An artificially synthesized primer sequence

<400>  5
gccctacgta gaccaaattt gcattggtta c                                    31

<210>  6
<211>  34
<212>  DNA
<213>  Artificial

<220>
<223>  An artificially synthesized primer sequence

<400>  6
atagtttagc ggccgcttat atttggtaag ttcc                                 34

<210>  7
<211>  33
<212>  DNA
<213>  Artificial

<220>
<223>  An artificially synthesized primer sequence

<400>  7
acgcgtcgac gcgcttcaag gcaccaaacg atc                                    33

<210>  8
<211>  30
<212>  DNA
<213>  Artificial

<220>
<223>  An artificially synthesized primer sequence

<400>  8
cccaagcttc tcctctgcat tgtctccgaa                                        30

<210>  9
<211>  18
<212>  DNA
<213>  Artificial

<220>
<223>  An artificially synthesized nucleotide sequence

<400>  9
gtcgttgtcg ttgtcgtt                                                     18

## Description

### Technical Field

The present invention relates to vaccination to poultry, to chickens in particular. Specifically, the present invention relates to immunization materials and methods for booster immunization targeting poultry subjected to an initial immunization with an injectable oil vaccine.

### Background Art

By 2008, influenza infecting birds was spreading in and around Southeast Asia. In particular, poultry farms sustained devastating damages (Bagchi, (2008) CMAJ, 178:1415). Furthermore, there were some human infections by the highly pathogenic subtypes of H5N1. A new influenza pandemic was feared (Wright, (2008) N. Engl. J. Med., 358:2540-2543). Under these circumstances, countermeasures against avian influenza virus became an important public health objective.

Induction of immunity by vaccination is an effective preventive measure for avian influenza. Hence, Japan has also produced and has a stockpile of an intramuscular injectable oil vaccine. If the immunity induced by this vaccination can be maintained at high levels over a long period, it will serve as a very effective preventive measure. However, conventional methods are insufficient in terms of effectiveness, cost, convenience, etc.

Methods for preparing materials for influenza vaccine include, for example, those described in "VACCINE USING SENDAI VIRUS VECTOR, AND VACCINE PROTEIN (Japanese Patent Application Kokai Publication No. (JP-A) 2000-253876 (unexamined, published Japanese patent application))", "RECOMBINANT INFLUENZA VIRUSES FOR VACCINES AND GENE THERAPY (JP-A (Kokai) 2003-528570)", and "METHOD FOR DEVELOPING PROTEIN VACCINES AND VACCINES FOR AVIAN INFLUENZA USING REVERSE GENETIC METHOD (JP-A (Kokai) 2007-282636)". However, none of the documents mentions booster immunization.

Meanwhile, A. D. Altstein *et al.* undertook an attempt at booster immunization with recombinant NP in mice, but the administration route was intraperitoneal administration (Altstein et al. (2006) Arch. Virol. 151: 921-931). G. Le Gall-Recule *et al.* also performed booster immunization with oil vaccine in chickens, but this administration route was subcutaneous inoculation (G. Le Gall-Recule et al. (2007) Avian Dis. 51: 490-494). Y. Asahi-Ozaki *et al.* prepared an HA vaccine using recombinant virus produced by reverse genetics, and they reported a result obtained by testing the vaccine with nasal administration (Y. Asahi-Ozaki et al. (2006) Microbes Infect. 8: 2706-2714).

As described above, there is no report of an effective case featuring an oral booster immunization method using recombinant protein antigens targeting chickens.

Prior art documents related to the present invention include:

### Prior Art Documents

### Patent Documents

Patent Document 1: JP-A (Kokai) 2000-253876
Patent Document 2: JP-A (Kokai) 2003-528570
Patent Document 3: JP-A (Kokai) 2007-282636

### Non-patent Documents

Non-patent Document 1: Bagchi, (2008) CMAJ, 178:1415
Non-patent Document 2: Wright, (2008) N. Engl. J. Med., 358:2540-2543
Non-patent Document 3: Altstein et al. (2006) Arch. Virol. 151: 921-931
Non-patent Document 4: G. Le Gall-Recule et al. (2007) Avian Dis. 51: 490-494
Non-patent Document 5: Y. Asahi-Ozaki et al. (2006) Microbes Infect. 8: 2706-2714
Non-patent Document 6: Eliasson D. G. et al. (2008) Vaccine 26:1243-52
Non-patent Document 7: Sylte M. J. et al. (2007) Vaccine 25:3763-72
Non-patent Document 8: Ohba K. et al. (2007) Vaccine 25:4291-300
Non-patent Document 9: Roy S. et al. (2007) Vaccine 25:6845-51
Non-patent Document 10: Huleatt J. W et al. (2008) Vaccine 26:201-14
Non-patent Document 11: Webster R. G. et al. (1991) Vaccine 9:303-8
Non-patent Document 12: Ilyinskii P. O. et al. (2008) Vaccine 26:2177-85
Non-patent Document 13: Zanvit P. et al. (2008) Immunol. Lett. 115:144-52
Non-patent Document 14: Isaka M. et al. (2008) Microbiol. Immunol. 52:55-63
Non-patent Document 15: Horthongkham N. et al. (2007) J. Immune. Based Ther. Vaccines 5:10

### Summary of the Invention

### [Problems to be Solved by the Invention]

An objective of the present invention is to provide immunization materials that enable longer maintenance of immunity induced by an initial immunization with an oil vaccine, at a high level, and to establish a convenient and efficient method for booster immunization.

### [Means for Solving the Problems]

Using recombinant DNA technology, the present inventors prepared HA and NP proteins, which are said to be antigens effective in preventing avian influenza. The mucosal adjuvant synthetic oligo CpG was combined with the recombinant HA and NP antigens and the mixture was orally administered to chickens that have been intramuscularly inoculated with an oil vaccine. The result showed that, even when the immune response induced by oil vaccine was impaired, antibody production was re-induced by oral administration of recombinant HA and NP antigens in combination with synthetic oligo CpG. The vaccine of the present invention was effective when used for booster immunization, while it was poorly effective or ineffective when used for initial immunization.

Specifically, the present invention provides:
[1] an immunization material comprising an avian influenza virus antigen and a mucosal adjuvant effective for oral administration;
[2] the immunization material of [1], wherein the avian influenza virus antigens are recombinant HA antigen and recombinant NP antigen;
[3] the immunization material of [1] or [2] for preventing avian influenza;
[4] the immunization material of any one of [1] to [3], which is used by oral administration;
[5] the immunization material of any one of [1] to [4], which is used for booster immunization;
[6] a method for re-inducing immunity induced by an initial immunization, wherein an immunization material of any one of [1] to [5] is additionally administered to an animal administered with an avian influenza vaccine;
[7] the method of [6], wherein the animal is a bird;
[8] the method of [6] or [7], wherein the booster administration is achieved by oral administration;
[9] use of the immunization material of [1] to [5] in manufacturing an agent for re-inducing immunity induced by initial immunization in an animal administered with vaccine against avian influenza; and
[10] use of the immunization material of [1] to [5] in a method for re-inducing immunity induced by initial immunization in an animal administered with vaccine against avian influenza.

### Brief Description of the Drawings

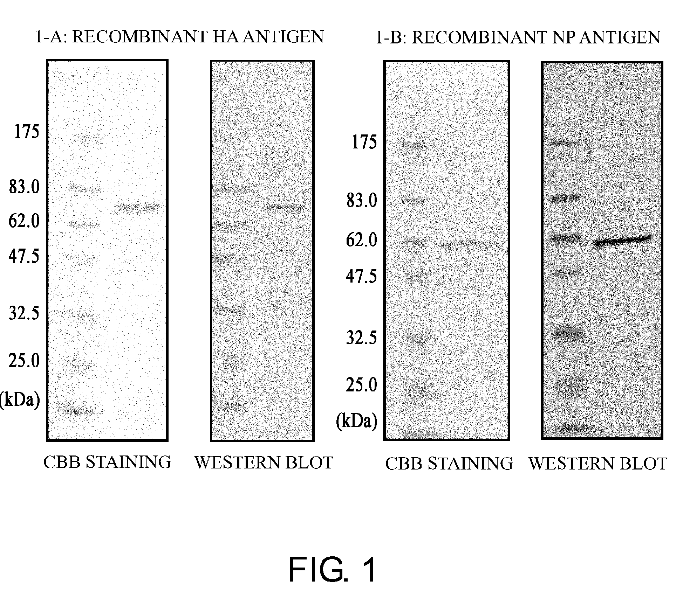

Fig. 1 shows photographs depicting antigen purity and reactivity to commercial antibodies confirmed by SDS-PAGE and Western blotting after purifying HA antigen (1-A) expressed by recombinant yeast containing the HA of A/duck/Mongolia/54/01 (H5N2) strain as a gene donor and NP antigen (1-B) expressed by recombinant *E. coli* containing the NP of A/duck/Hokkaido/9/99 (H9N2) strain as a gene donor.

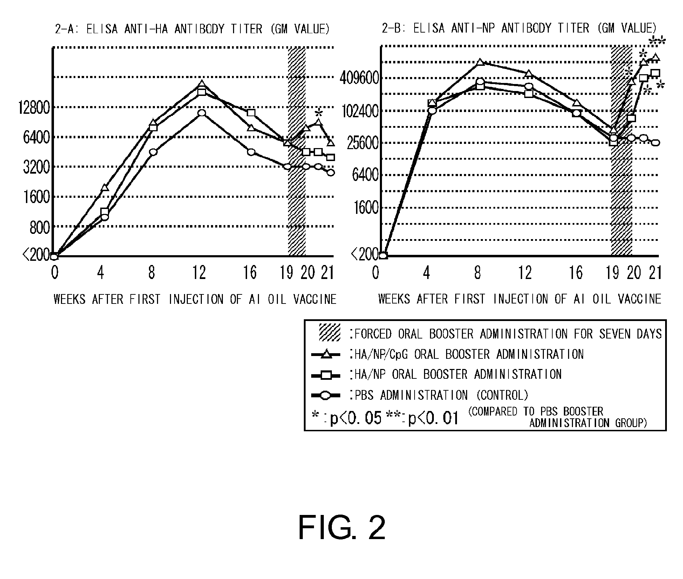

Fig. 2 shows graphs depicting quantified and plotted time courses of titers of anti-HA and anti-NP antibodies before and after oral administration of recombinant HA and NP antigens, and mucosal adjuvant to chickens inoculated with an oil vaccine against avian influenza.

### Mode for Carrying Out the Invention

The present invention provides immunization materials containing avian influenza virus antigens and a mucosal adjuvant that is effective for oral administration. The immunization materials of the present invention are preferably used to prevent influenza.

Influenza is a life-threatening serious infection in patients with pre-existing diseases or aged persons. Furthermore, there have recently been epidemics of avian influenza, which is infection in birds. Among type A influenza virus infections, highly pathogenic avian influenza exhibits markedly strong pathogenicity in poultry such as chicken, quail, and turkey. The highly pathogenic avian influenza is defined as an avian influenza virus that kills chickens at a high probability when inoculated into the vein, or as a so-called highly-virulent virus having consecutive basic amino acids at the cleavage site in HA.

Type A influenza viruses are classified based on the type of HA (and type of NA). Highly pathogenic avian influenza viruses identified to date are limited to subtypes H5 and H7. These subtype viruses can mutate into highly-virulent forms after repeated cycles of infection. Thus, precaution should be taken against these viruses regardless of whether they are in the highly virulent form or less virulent form.

Influenza viruses that are originally non-infectious in humans, for example, subtype viruses H5 and H7, can mutate to become infectious and capable of propagating in humans. Such mutant viruses that have finally acquired the ability to cause infection at a high frequency from humans to humans are called new influenza viruses. Since humans have no immunity against such new influenza viruses, the infection can spread and may cause a world-wide epidemic (pandemic), with prospects of causing tremendous damage.

"Influenza virus antigen" refers to a recombinant antigen originating from influenza virus as gene donor, which has an ability to prevent the infection and onset of influenza. Influenza virus antigens that are already known to have the effect of preventing influenza include, for example, hemagglutinin (HA) antigen, neuraminidase (NA) antigen, NP (nuclear protein) antigen, and M protein (matrix protein) antigen.

An influenza virus antigen to be contained in an immunization material of the present invention may be any subtype virus as long as it allows oral booster immunization to re-induce the immunity or to produce the effect of resisting immunity impairment. Antigens applicable to the present invention include, but are not limited to, for example, (1) HA and NA, which are viral surface antigens, and (2) NP and M, which are antigens located in the internal structure of a virus. PA, PB1, PB2, and NS2 can also be used in the present invention. They may be used alone or in any combination of two or more.

Preferred influenza virus antigens to be contained in an immunization material of the present invention are HA antigen and NP antigen. The influenza virus antigens of the present invention are not limited to the above-described antigens. Spontaneous or artificially produced mutant antigens having an altered gene sequence or amino acid sequence are also included in the present invention, as long as they are antigens that are functionally equivalent to the HA or NP antigen. Herein, an antigen functionally equivalent to HA antigen refers to a protein having the same antigenicity as HA antigen. As used herein, an antigen functionally equivalent to NP antigen refers to a protein having the same antigenicity as NP antigen.

An influenza virus antigen to be contained in an immunization material of the present invention is preferably a recombinant HA antigen. Such recombinant HA antigens include, for example:
(a) a protein comprising the amino acid sequence of SEQ ID NO: 2;
(b) a protein encoded by a nucleic acid comprising the coding region having the nucleotide sequence of SEQ ID NO: 1;
(c) a protein comprising an amino acid sequence with a substitution, deletion, insertion, and/or addition of one or more amino acids in the amino acid sequence of SEQ ID NO: 2, which is functionally equivalent to a protein comprising the amino acid sequence of SEQ ID NO: 2; and
(d) a protein encoded by a nucleic acid that hybridizes under stringent conditions to a nucleic acid comprising the nucleotide sequence of SEQ ID NO: 1, which is functionally equivalent to a protein comprising the amino acid sequence of SEQ ID NO: 2.

An influenza virus antigen to be comprised in an immunization material of the present invention is preferably a recombinant NP antigen. Such recombinant NP antigens include, for example:
(a) a protein comprising the amino acid sequence of SEQ ID NO: 4;
(b) a protein encoded by a nucleic acid comprising the coding region having the nucleotide sequence of SEQ ID NO: 3;
(c) a protein comprising an amino acid sequence with a substitution, deletion, insertion, and/or addition of one or more amino acids in the amino acid sequence of SEQ ID NO: 4, which is functionally equivalent to a protein comprising the amino acid sequence of SEQ ID NO: 4; and
(d) a protein encoded by a nucleic acid that hybridizes under stringent conditions to a nucleic acid comprising the nucleotide sequence of SEQ ID NO: 3, which is functionally equivalent to a protein comprising the amino acid sequence of SEQ ID NO: 4.

Known methods which cause random mutations to a given nucleotide sequence include substitution(s) of base pair(s) through nitrous acid treatment of DNA (Hirose, S. et al., Proc. Natl. Acad. Sci. USA., 79:7258-7260, 1982). This method enables random introduction of substitution(s) of base pair(s) into a specific segment by nitrous acid treatment of the segment desired to be mutated. Alternatively, technologies for site-directing a target mutation include the gapped duplex method (Kramer W. and Fritz HJ., Methods in Enzymol., 154:350-367, 1987) and the like. A cyclic double stranded vector in which a gene to be introduced with a mutation is cloned, is separated into single strands. These single strands are hybridized with a synthetic oligonucleotide mutated at the target site. A vector-derived complementary single strand DNA cleaved and linearized by a restriction enzyme is annealed with the cyclic single stranded vector, and the gap between the oligonucleotide and the vector is filled by using a DNA polymerase, which is then made into a complete double stranded vector by ligation.

The number of amino acids to be modified would be typically within 50 amino acids, preferably within 30 amino acids, and more preferably within 5 amino acids (for example, 1 amino acid).

When an amino acid is artificially substituted, substitution with an amino acid having similar properties would result in maintaining the activity of the original protein. Proteins of the present invention include proteins resulting from a conservative substitution in the above substitution of amino acid(s), and which are functionally equivalent to the proteins comprising the amino acid sequence of SEQ ID NO: 2 or 4. Conservative substitution is considered important when substituting amino acid(s) of domains important for protein activities, etc. Such a conservative substitution of amino acid(s) is well known to those skilled in the art.

Examples of amino acid groups suitable for conservative substitution include basic amino acids (such as lysine, arginine, and histidine), acidic amino acids (such as aspartic acid and glutamic acid), uncharged polar amino acids (such as glycine, asparagine, glutamine, serine, threonine, tyrosine, and cysteine), nonpolar amino acids (such as alanine, valine, leucine, isoleucine, proline, phenylalanine, methionine, and tryptophane), β branched amino acids (such as threonine, valine, and isoleucine), and aromatic amino acids (such as tyrosine, phenylalanine, tryptophane, and histidine).

Moreover, non-conservative substitution may increase protein activities or such (for example, constitutively activated proteins).

In addition, proteins which are functionally equivalent to the proteins comprising the amino acid sequence of SEQ ID NO: 2 or 4 can be obtained by methods that utilize hybridization. That is to say, a DNA encoding an influenza virus antigen of the present invention as shown in the SEQ ID NO: 1 or 3, or a fragment thereof is used as a probe, and then DNAs that can hybridize to them are isolated. A hybridization reaction performed under stringent conditions leads to the selection of highly homologous DNA as a nucleotide sequence. This increases the chances of isolated proteins containing proteins that are functionally equivalent to the influenza virus antigen. Examples of a highly homologous nucleotide sequence include those having 70% or more, and desirably 90% or more homology (identity).

In a specific example, the term "stringent conditions" refers to hybridization conditions with 6 x SSC, 40% formamide at 25°C and subsequent washing with 1x SSC at 55°C. The stringency depends on conditions such as salt concentration, formamide concentration, or temperature; however it is apparent for those skilled in the art to set these conditions so as to obtain a necessary stringency.

With the use of hybridization, for example, DNAs encoding homologues of the influenza virus antigens other than those proteins comprising the amino acid sequence of SEQ ID NO: 2 or 4 can be isolated.

Proteins which are functionally equivalent to a protein comprising the amino acid sequence of SEQ ID NO: 2 or 4 normally have a high homology with the amino acid sequence of SEQ ID NO: 2 or 4. The term "high homology" refers to a sequence homology (identity) of at least 30% or more, preferably 50% or more, more preferably 80% or more (for example, 95% or more). The homology of the nucleotide sequences and amino acid sequences can be determined using a homology search site via the internet (For example, homology searches such as FASTA, BLAST, PSI-BLAST, and SSEARCH can be used in the DNA Data Bank of Japan (DDBJ) [examples of which include the homology search page (Search and Analysis) at the DNA Data Bank of Japan (DDBJ) website; http://www.ddbj.nig.ac.jp/E-mail/homology-j.html]. Furthermore, searches using BLAST can be carried out through the web site of the National Center for Biotechnology Information (NCBI) [examples of which include BLAST page at the homepage of NCBI website; http://www.ncbi.nlm.nih.gov/BLAST/; Altschul, S.F. et al., J. Mol. Biol., 1990, 215(3):403-10; Altschul, S.F. & Gish, W., Meth. Enzymol., 1996, 266:460-480; Altschul, S.F. et al., Nucleic Acids Res., 1997, 25:3389-3402]).

For example, in the calculation of the identity of amino acid sequences using Advanced BLAST 2.1, the identity value (%) can be obtained by the following: blastp is used as the program, expect value is set at 10, all filters are set at OFF, BLOSUM62 is used for matrix, and gap existence cost, per residue gap cost, and lambda ratio are set at 11, 1, and 0.85, respectively (default parameters) (Karlin, S. and S. F. Altschul (1990) Proc. Natl. Acad. Sci. USA 87:2264-68; Karlin, S. and S. F. Altschul (1993) Proc. Natl. Acad. Sci. USA 90:5873-7).

The recombinant antigens of the present invention are not particularly limited, as long as they are suitable for oral administration. The antigens include, for example, the antigens expressed by recombinant DNA technologies in unicellular prokaryotes such as *E. coli* or unicellular eukaryotes such as yeast, and the antigens expressed in multicellular organisms such as insect, plant, and animal cells.

When eukaryotic cells are used, host cells include, for example, animal cells, plant cells, and fungal cells. Known animal cells include mammalian cells such as CHO (J. Exp. Med. 108, 945 (1995)), COS, 3T3, myeloma, BHK (baby hamster kidney), HeLa, and Vero; amphibian cells such as *Xenopus laevis* oocytes (Valle, et al., Nature 291, 358-340 (1981)); and insect cells such as Sf9, Sf21, and Tn5. In the present invention, CHO-DG44, CHO-DXB11, COS7, and BHK cells are preferably used. Of the animal cells, CHO cells are particularly preferable for large-scale expression. Vectors can be introduced into host cells, for example, by calcium phosphate methods, DEAE-dextran methods, methods using cationic liposome DOTAP (Boehringer-Mannheim), electroporation methods, lipofection methods, etc.

It is known that plant cells such as *Nicotiana tabacum-derived* cells are protein production systems, and callus cultures from these cells may be used. Protein systems using fungal cells including yeasts, for example, the genus *Saccharomyces* such as *Saccharomyces cerevisiae* and *Saccharomyces pombe*; and filamentous fungi, for example, the genus *Aspergillus* such as *Aspergillus niger* are known.

When prokaryotic cells are used, production systems that use bacterial cells are available. Such bacterial cells include *E. coli*, for example, JM109, DH5α, and HB101, and *Bacillus subtilis*. An antigen of the present invention can be prepared by culturing cells transformed with a DNA of the present invention *in vitro* and purifying the resulting antigen by methods normally used by those skilled in the art.

"Mucosal adjuvant" refers to a substance that enhances the immune response when administered to mucosa. An immune adjuvant may also be referred to as an adjuvant, adjuvant for vaccination, vaccination adjuvant, vaccine adjuvant, immune adjuvant composition, adjuvant composition, composition for adjuvant, immunostimulant, or immunopotentiator. All of these substances refer to the immune adjuvant of the present invention. Mucosal adjuvants included in the immunization materials of the present invention include, but are not limited to, for example, synthetic oligo CpG (Ameiss et al., (2006) Vet. Immunol. Immunopathol. 110:257-67), lectin, liposomes, *E. coli* heat labile toxin (or cholera toxin), and interleukin-12. A preferred mucosal adjuvant to be contained in an immunization material of the present invention is synthetic oligo CpG.

The present invention provides methods for re-inducing the immunity induced by initial immunization in animals administered with vaccine against avian influenza, by additionally administering an immunization material containing avian influenza virus antigens and a mucosal adjuvant effective in oral administration. Thus, the immunization material of the present invention is used for booster immunization.

Immunization materials of the present invention exert their effects when administered (booster immunization) to individuals in which the antibody titer has been already elevated by vaccination (for example, administration by injection). The administered immunization materials can further elevate the antibody titer or maintain it at high levels in the individuals. For example, even when a high-level antibody titer already begins to drop in individuals inoculated with oil vaccine in initial immunization, the antibody titer can be significantly re-elevated in the individuals by orally administering recombinant antigens and a mucosal adjuvant.

The vaccine efficacy can be assessed by antibody titer assay using ELISA, HI titer assay, neutralization antibody titer assay, or clinical observation or survival assessment in viral attack test using mice or the like. These methods are known to those skilled in the art.

Subject animals to be administered with immunization materials of the present invention include, for example, humans, monkeys, birds, horses, and pigs. Preferred subjects to be administered with immunization materials of the present invention are birds.

The administration route for the immunization materials of the present invention is mucosal administration, and a preferred route is oral administration.

Immunization materials of the present invention can be formulated according to the usual methods (for example, Remington's Pharmaceutical Science, latest edition, Mark Publishing Company, Easton, U.S.A), and may contain pharmaceutically acceptable carriers and additives together. Examples include surfactants, excipients, coloring agents, preservatives, stabilizers, buffers, suspending agents, and isotonizing agents, although they are not limited thereto and other common carriers may be appropriately used.

A recombinant antigen prepared using as a gene donor an influenza virus of the present invention may be administered orally after formulating it in combination with other ingredients. Such ingredients used in combination include, for example, feed for the target animal, flavor-improving additives (lactose, etc.), and drinking water.

The dose varies depending on the type of animal which receives administration. For example, when a recombinant antigen derived from influenza virus is used in crude or partially purified form, chickens are conveniently fed a diet prepared by mixing ordinary feed with the antigen at a weight ratio of around 5% ("ORAL VACCINE AGAINST PROTOZOIASIS USING GENETICALLY MODIFIED PLANT (International Application No. WO 2005/116216)").

The single dose can be selected in the range of 1 to 100 mcg (microgram; the same abbreviation is used hereinafter) of purified protein per bird, when the antigen is administered, for example, three to 14 times for three to 14 days. However, the dose is not limited to such doses. The antigen may be administered consecutively or at any interval.

For other animals, the antigen can be administered at a dose determined based on the body weight or body surface area. However, the dose is not limited to such doses. The appropriate dose can be determined by considering the expression level of the protective antigen, optimal antibody production, properties of formulated feed, and the like.

Use of immunization materials of the present invention may be described as follows:
(1) use of an immunization material comprising avian influenza virus antigens and a mucosal adjuvant effective in oral administration in manufacturing agents for re-inducing the immunity induced by initial immunization in animals administered with vaccine against avian influenza; or
(2) use of an immunization material comprising avian influenza virus antigens and a mucosal adjuvant effective in oral administration in methods for re-inducing the immunity induced by initial immunization in animals administered with vaccine against avian influenza.

All prior art documents cited in the specification are incorporated herein by reference.

### Examples

The present invention is specifically illustrated below with reference to Examples, but it is not to be construed as being limited thereto.

### [Example 1] Preparation of mucosal adjuvant and recombinant antigen proteins originating from influenza virus

### (1) Preparation of recombinant HA antigen

The gene encoding the amino acids of positions 17 to 528 relative to the N terminus in the HA protein of A/duck/Mongolia/54/01 (H5N2) strain was amplified by reverse transcription-PCR.

Specifically, reverse transcription-PCR was carried out using One Step RNA PCR kit (AMV) (TaKaRa) with primers 5'-GCC CTA CGT AGA CCA AAT TTG CAT TGG TTA C-3' (SEQ ID NO: 5) and 5'-ATA GTT TAG CGG CCG CTT A TAT TTG GTAAGT TCC-3' (SEQ ID NO: 6).

The resulting cDNA fragment (nucleotide sequence/SEQ ID NO: 1; amino acid sequence/SEQ ID NO: 2) was digested with restriction enzymes *Sna*BI and *Not*I, and then inserted into plasmid pPIC9K (Multi-Copy Pichia Expression Kit; Invitrogen). The constructed expression plasmid for HA was introduced into *Pichia pastoris* GS 115 strain (Multi-Copy Pichia Expression Kit; Invitrogen). This transformation experiment was conducted according to the instruction manual appended to the Multi-Copy Pichia Expression Kit.

A clone expressing recombinant HA was selected from the obtained transformants by Western blotting using Rabbit Polyclonal anti-Avian Influenza A Hemagglutinin antibody (Novus) and Goat anti-Rabbit IgG-heavy and light chain cross-adsorbed Antibody HRP Conjugated (Bethyl Laboratories).

The obtained HA-expressing yeast was inoculated to YPD medium (2% glucose, 1% yeast extract, 2% peptone) and cultured at 28°C with shaking overnight. The culture medium was inoculated to MGY medium (1% glycerol, 0.00004% biotin, yeast nitrogen base (Invitrogen)), and the yeast was cultured at 28°C with shaking until OD₆₀₀ reached 2.0 from 1.5 (for 16 to 18 hours). The culture medium was aseptically centrifuged (2,000 x g for five minutes) to harvest the microbial cells. The resulting yeast cells were re-suspended in BMMY medium (0.5% methanol, 1% yeast extract, 2% peptone, 0.00004% biotin, yeast nitrogen base (Invitrogen), 100 mM phosphate buffer (pH6.0)). This suspension was incubated at 28°C with shaking for 16 hours, and then methanol (0.5% of the culture medium volume) was aseptically added. After six hours of culture, the obtained yeast cells were harvested by centrifugation at 5,000 x g for 15 minutes, and frozen at -80°C. The cells were thawed and re-suspended in breaking buffer (50 mM Tris-Cl, 1 mM EDTA, 5% glycerol (pH 8.0)). Glass beads (425 to 600 microns; Sigma) were added to the suspension. The cells were crushed in Bioruptor (Cosmo bio) by three rounds of 30 cycles of sonication for 30 seconds and resting for 30 seconds. The extract was centrifuged (14,000 x g for 30 minutes), and the supernatant was collected and clarified using a filter with 0.22-mcm (micrometer, the same abbreviation is used hereinafter) pores. The resulting sample was used as crude extract.

The crude extract was fractionated in HiPrep 16/10 Q XL column (GE healthcare). The binding buffer used was 20 mM Tris-Cl (pH 8.0). Elution was achieved using an elution buffer of 20 mM Tris-Cl (pH 8.0) with an NaCl concentration gradient up to 1M. Fractions containing recombinant HA were selected by Western blotting using the same antibodies described above.

The collected fractions were concentrated and dialyzed by ultrafiltration (cut-off molecular weight, 30 kDa). The buffer was replaced with 20 mM Tris-Cl (pH 8.0) containing 150 mM NaCl. The purity of the prepared recombinant HA antigen was confirmed by 10% SDS-PAGE and Western blotting (Fig. 1 (1-A)).

### (2) Preparation of recombinant NP antigen

The NP gene was prepared from A/duck/Hokkaido/9/99 (H9N2) strain by reverse transcription-PCR using One Step RNA PCR kit (AMV) (TaKaRa) with 5'-ACG CGT CGA CGC GCT TCA AGG CAC CAA ACG ATC-3' (SEQ ID NO: 7) and 5'-CCC AAG CTT CTC CTC TGC ATT GTC TCC GAA-3' (SEQ ID NO: 8).

The cDNA fragment (nucleotide sequence/SEQ ID NO: 3; amino acid sequence/SEQ ID NO: 4) of the NP gene was digested with restriction enzymes *Sal*I and *Hind*III, and inserted into plasmid vector pQE31 (Qiagen) for expressing a recombinant protein in *E. coli.* The recombinant DNA was introduced into *Escherichia coli* M15 strain to prepare recombinant *E. coli* expressing recombinant NP. This recombination experiment was conducted according to the instruction manual provided by Qiagen.

The obtained recombinant NP-expressing *E. coli* was inoculated in LB medium (1.0% tryptone, 0.5% yeast extract, 1.0% sodium chloride, 100 mcg/ml ampicillin sodium salt, 25 mcg/ml kanamycin sulfate), and cultured at 37°C with shaking overnight. This pre-culture was inoculated in a 100-times larger volume of fresh LB medium. The medium was incubated with shaking at 37°C until OD₆₀₀ reached 0.6. To grow the bacteria until the turbidity reached a desired level, IPTG was added at a final concentration of 1 mM to the medium and the bacteria were incubated at 37°C with shaking overnight to induce the expression of NP protein. After culture, the bacterial cells were harvested by centrifugation at 5,000 x g for 20 minutes. The bacterial cells were re-suspended in 20 mM Tris-HCl buffer (pH 8.0), and lysozyme, a bacteriolysis enzyme, was added thereto at a final concentration of 0.4 mg/ml. The suspension was incubated on ice for 30 minutes for bacteriolysis. After the reaction, sodium deoxychlorate was added at a final concentration of 0.1%. The mixture was incubated at 37°C for 30 minutes. After treating the bacterial lysate with Polytron (8,000 rpm for two minutes), the lysate was centrifuged at 10,000 x g for 30 minutes.

50 ml of the resulting extract was combined with 5 ml of Ni-NTAAgarose (Qiagen). The mixture was incubated at 4°C for one hour for binding, and loaded onto a polypropylene column (5 ml). The column was washed with 20 ml of washing buffer A (20 mM Tris-HCl, 0.5 M NaCl, 20 mM imidazole (pH 8.0)) and washing buffer B (20 mM Tris-HCl, 0.5 M NaCl, 75 mM imidazole (pH 8.0)). Then, 2.5 ml of elution buffer (20 mM Tris-HCl, 0.5 M NaCl, 250 mM imidazole (pH 8.0)) was loaded four times onto the column to elute recombinant NP. The obtained antigen protein was used after dialyzing against 20 mM Tris-HCl (pH8.0) buffer containing 0.5 M NaCl. The purity of the prepared recombinant NP antigen was confirmed by 10% SDS-PAGE and Western blotting (Fig. 1 (1-B)).

### (3) Preparation of mucosal adjuvant

The CpG sequence 5'-GTC GTT GTC GTT GTC GTT-3' (Ameiss et al., (2006) Vet. Immunol. Immunopathol. 110:257-67; SEQ ID NO: 9) which has been reported to produce the effect of enhancing the immunity when used as a mucosal adjuvant by oral administration was used in the present invention. The DNA (CpG-oligo dinucleotide; hereinafter abbreviated as CpG-ODN) was designed to have sulfur atoms in the phosphate backbone instead of oxygen atoms to enhance the resistance to degradation. The synthesis of the DNA was ordered to Operon Co.

After ethanol precipitation, the synthesized CpG-ODN was dissolved in sterile PBS (137 mM NaCl, 8.1 mM Na₂HPO₄, 2.68 mM KC1, 1.47 mM KH₂PO₄) and stored at -20°C until use in experiments.

### [Example 2] Oral administration of recombinant antigens and mucosal adjuvant to chickens inoculated with oil vaccine

An oil vaccine against avian influenza was inoculated to four-week-old white leghorns (line-M) as initial immunization. Specifically, 10⁴ TCID50 of virus was prepared using avian influenza virus A/duck/Hokkaido/Vac-1/04 (H5N1) strain as seed virus, and 0.2-ml aliquots of the virus were each inoculated into the allantoic cavities of 11-day-old embryonated hen eggs. The eggs were incubated at 34°C for 72 hours, and then allowed to stand at 4°C overnight. The allantoic fluid was harvested from the hen eggs after infection, and centrifuged (3,000 x g for 20 minutes) to obtain the supernatant. After adding β-propiolactone at a final concentration of 0.05% (v/v), to inactivate the virus, the supernatant was incubated at 4°C for five days. The inactivated viral suspension was assayed for the HA titer, and then diluted to 5,120 HAU/ml with PBS. The resulting suspension was used as an antigen solution. Light liquid paraffin, tetraoleic acid polyoxyethylene (40) sorbitol, and sorbitan sesquioleate were added at concentrations of 65%, 1.4% (w/v), and 2.0% (w/v), respectively, to the antigen solution. The mixture was emulsified with an emulsifier device (circulation flow volume of 4 1/min, 8,000 rpm, 0.5 kPa) to give a vaccine. The HA titer of the oil vaccine was 1,024 HAU/ml. 0.5 ml of the vaccine was intramuscularly inoculated to each chicken. Specifically, the dose of antigen per bird was 512 HAU.

The immune response induced by the vaccination in the subject chickens was monitored by venous blood sampling at one-week intervals, followed by ELISA for the antibody titer. Specifically, recombinant HA or NP antigen was diluted to 0.1 mcg/ml with 0.05 M sodium carbonate buffer (1.59 g/l Na₂HCO₃, 2.93 g/l NaH₂CO₃ (pH 9.6)), and aliquoted in 96-well ELISA plates (IWAKI). The plates were allowed to stand at 4°C overnight for coating. After the plates were washed with PBS-T buffer (0.05% Tween20, 137 mM NaCl, 8.1 mM Na₂HPO₄, 2.68 mM KC1, 1.47 mM KH₂PO₄) a blocking solution prepared by adding bovine serum albumin at a final concentration of 3% (w/v) to PBS-T buffer was aliquoted into the plates. The plates were allowed to stand at 37°C for one hour for blocking. After blocking treatment, the plates were washed with PBS-T buffer. Then, bovine serum albumin was added at a final concentration of 0.3% (w/v) to PBS-T buffer to prepare an antibody dilution buffer. The sera of the subj ect chickens were diluted 100 fold up to 51,200 fold by serial twofold dilution and aliquoted into the plates. The plates were allowed to stand at 37°C for one hour for reaction with antigen. After the plates were washed with PBS-T buffer, HRPO-labeled anti-chicken IgG antibody (ZYMED) diluted 12,000 fold with the antibody dilution buffer was aliquoted into the plates. The plates were incubated at 37°C for one hour. After the plates were washed with PBS-T buffer, a substrate solution (14.6 g/l Na₂HPO₄, 10.2 g/l citrate monohydrate, 1 g/l ortho-phenylenediamine, 1 ml/l hydrogen peroxide) was added thereto. The plates were allowed to stand at 37°C in the dark for 15 minutes. A termination solution (5N sulfuric acid) was added to stop the reaction. The absorbance at 492 nm was measured in a microplate reader (Corona MTP-120).

Recombinant HA and NP antigens and mucosal adjuvant CpG-ODN were orally administered to the chickens (during 19th week after immunization with oil vaccine) whose ELISA antibody titers dropped after the maximal level. Specifically, the following three groups were prepared: (i) a group administered with 100 mcg of recombinant HA, 100 mcg of recombinant NP, and 25 mcg of CpG-ODN; (ii) a group administered with 100 mcg of recombinant HA and 100 mcg ofrecombinant NP; and (iii) a group administered with PBS. Each group consisted of four chickens. Each antigen and CpG-ODN were adjusted to a total volume of 2 ml with PBS, and administered orally with a forced water load using a syringe. They were administered every day for consecutive seven days. The ELISA antibody titer was monitored over time during the administration period and one week after administration.

The experiment result is shown in Fig. 2.

This experiment result showed that, even when the immune response induced with oil vaccine was impaired, the antibody titer could be re-induced by orally administering the recombinant HA and NP, and CpG-ODN in combination (Fig. 2; p<0.05). Marked re-induction of immunity was observed particularly against NP, an antigen shared by all subtypes of type A influenza (Fig. 2 (2-B); p<0.01). The vaccine of the present invention was effective when used for booster immunization, while it was poorly effective or ineffective when used for initial immunization.

### Industrial Applicability

The present invention provides immunization materials and booster immunization methods that enable longer maintenance of immunity induced by an initial immunization with an oil vaccine, at a high level. The immunization materials and booster immunization methods of the present invention enable convenient and efficient vaccination for preventing influenza.

## Claims

1. An immunization material comprising an avian influenza virus antigen and a mucosal adjuvant effective for oral administration.

2. The immunization material of claim 1, wherein the avian influenza virus antigens are recombinant HA antigen and recombinant NP antigen.

3. The immunization material of claim 1 or 2 for preventing avian influenza.

4. The immunization material of any one of claims 1 to 3, which is used by oral administration.

5. The immunization material of any one of claims 1 to 4, which is used for booster immunization.

6. A method for re-inducing immunity induced by an initial immunization, wherein an immunization material of any one of claims 1 to 5 is additionally administered to an animal administered with an avian influenza vaccine.

7. The method of claim 6, wherein the animal is a bird.

8. The method of claim 6 or 7, wherein the booster administration is achieved by oral administration.
